# EUROPEAN PATENT APPLICATION

(11) **EP 4 385 506 A1**
(43) Date of publication of application: **19.06.2024**
(21) Application number: 22855796.3
(22) Date of filing: 27.07.2022
(51) Int. Cl.: A61K 31/198, A61K 8/44, A61K 9/08, A61K 47/18, A61K 47/22, A61K 47/26, A61P 17/00, A61P 17/16, A61P 17/18, A61Q 17/00

(54) **STABILIZATION OF MYCOSPORINE-LIKE AMINO ACID**

(30) Priority: 12.08.2021 JP 2021131709
(71) Applicant: Nagase & Co., Ltd., Osaka 550-8668 (JP)
(72) Inventor: YAMAMOTO, Shogo, Kobe-shi, Hyogo 651-2241 (JP)
(74) Representative: dompatent von Kreisler Selting Werner - Partnerschaft von Patent- und Rechtsanwälten mbB
(86) International application number: PCT/JP2022/028940
(87) International publication number: WO 2023/017733

(57) **Abstract**

Provided are: a MG-stabilized composition which comprises mycosporine-glycine (MG) and is acidic to weakly alkaline; an MG-stabilized composition which comprises MG and at most 10 parts by weight of 4-DG; and an MAA-stabilized composition which comprises MAA and at least one selected from a base constituting a nucleic acid, a nucleoside, a glycine, and a tranexamic acid.

## Description

### [Technical Field]

The present invention relates to a composition in which a mycosporine-like amino acid (hereinafter referred to as "MAA") is stabilized, and a method for stabilizing MAA.

Ultraviolet rays (UV) are known to induce acute skin reactions such as erythema, skin aging and skin cancer. Ultraviolet rays contained in sunlight are classified into three types depending on their wavelength: UV-A (320 nm to 400 nm), UV-B (280 nm to 320 nm), and UV-C (200 to 280 nm). Among these, UV-A and UV-B have an effect on living organisms, while UV-C does not normally pose a problem because it cannot pass through the atmosphere.

UV-B is the main cause of sunburn outdoors and is known to have relatively higher energy than UV-A. When absorbed into the skin layer, it reaches the stratum corneum and epidermis, causing acute skin pigmentation such as age spots and freckles. UV-B is also known to cause immunosuppression, which is associated with aging and the development of skin cancer.

Although UV-A has lower energy than UV-B, it has a longer wavelength, and is known to penetrate deeper into the skin than UV-B, reaching the dermis. As a result, it causes not only acute skin pigmentation such as age spots and freckles, but also a decrease in the elasticity of the dermis (solar elastosis), causing to premature skin aging phenomena such as wrinkles and sagging. Furthermore, in recent years, it has been found that UV-A also causes immunosuppression and is involved in the development of precancerous skin lesions and skin cancer.

While the amount of UV-B varies depending on the season, weather, latitude, etc., a constant amount of UV-A reaches the earth's surface throughout the year. Therefore, it is important to protect the skin not only from UV-B but also from UV-A.

Current UV protection agents can be classified into UV absorbers and UV scattering agents. Ultraviolet absorbers convert ultraviolet energy into heat energy and emit it, and include, for example, a synthetic organic compound such as 4-tert-butyl-4'-methoxydibenzoylmethane. Ultraviolet scattering agents contain inorganic particles such as titanium oxide (TiO2) and zinc oxide (ZnO), and when applied to the skin, the inorganic particles present on the skin surface function as a barrier that reflects ultraviolet rays.

Ultraviolet absorbers have problems: (1) they are easily decomposed by light and have poor stability; (2) they cause molecular excitation and promote melanin production, causing itching and allergies; and (3) they give a bad image to users because they are chemically synthesized substances. Ultraviolet scattering agents have problems: (1) they tend to whiten the skin and give a heavy feeling when applied to the skin, (2) they cause the production of reactive oxygen species, and (3) they block the pores of the skin and there is a concern of inhibiting the function of exocrine glands. Due to these problems, expectations are increasing for safe, naturally-derived ultraviolet absorbing substances.

MAA is a natural UV absorbing substance and is known to be widely present in aquatic organisms such as corals, red algae, fish internal organs, and microalgae. MAA has a high ability to absorb UV-A and UV-B. MAA also has excellent antioxidant function. Therefore, MAA is used in cosmetics and skin compositions.

MAA is thus an extremely useful substance. On the other hand, since MAA has the problem of being easily decomposed and the decomposed product being colored, studies have been conducted on stabilizing MAA (Patent Document 1).

### [Citation list]

### [Patent documents]

[Patent document 1] JP 2017-197475 A

### [Summary of Invention]

### [Problem to be solved by the invention]

It is necessary to stabilize MAA and enhance its utility.

### [Means to solve the Problem]

The present inventors studied variously on substances and conditions for stabilizing MAA, found substances and conditions suitable for stabilizing MAA, and completed the present invention.

That is, the present invention provides:
(1) A composition in which mycosporine-glycine (MG) is stabilized, wherein the composition comprises MG and is weakly acidic to weakly alkaline.
(2) The composition of (1), pH of which is 5.4 to 8.0.
(3) A method for stabilizing MG, comprising subjecting MG under weakly acidic to weakly alkaline conditions.
(4) The method of (3), wherein pH is 5.4 to 8.0.
(5) A composition in which MG is stabilized, wherein the composition comprises:
   (a) MG, and
   (b) 10 parts by weight or less of 4-deoxygadusol (4-DG).
(6) The composition of (5), comprising 1.5 parts to 10 parts by weight of 4-DG.
(7) The composition of (5) or (6), which is weakly acidic to weakly alkaline.
(8) The composition of (7), pH of which is 5.4 to 8.0.
(9) A method for stabilizing MG, comprising mixing MG with 10 parts by weight or less of 4-DG.
(10) A composition in which MAA is stabilized, wherein the composition comprises:
   (a) mycosporine-like amino acid (MAA), and
   (b) one or more selected from a base constituting a nucleic acid, a nucleoside, glycine, and tranexamic acid.
(11) The composition of (10), wherein MAA is one or more MAA selected from MG, shinorine, mycosporine-glycine-alanine, and porphyra-334.
(12) The composition of (10) or (11), wherein MAA is one or more MAA selected from MG, mycosporine-glycine-alanine, and shinorine.
(13) The composition of any one of (10) - (12), which is weakly acidic to weakly alkaline.
(14) The composition of (13), pH of which is 5.4 to 8.0.
(15) A method for stabilizing MAA, comprising mixing MAA with one or more selected from a base constituting a nucleic acid, a nucleoside, glycine, and tranexamic acid.
(16) The method of (15), wherein MAA is one or more MAA selected from MG, shinorine, mycosporine-glycine-alanine, and porphyra-334.
(17) The method of (15) or (16), wherein MAA is one or more MAA selected from MG, mycosporine-glycine-alanine, and shinorine.

### [Effect of the invention]

According to the present invention, a composition in which MAA is stabilized and a method for stabilizing MAA are provided. The present invention is applicable to a wide range of MAA and can increase their value. By using the present invention and MAA, it is possible to produce cosmetics, skin compositions, etc. that have long-lasting effects such as UV absorption ability and have excellent preservability.

### [Brief description of drawings]

[Figure 1] The left panel of Figure 1 is a graph showing the results of investigating the influence of pH on the stability of MG. The right panel of Figure 1 is a graph showing the results of investigating the influence of pH on the coloring of the MG solution.
[Figure 2] The upper panel of Figure 2 is a graph showing the results of investigating the influence of the 4-DG ratio on the stability of MG. The lower panel of Figure 2 is a table showing the results of investigating the influence of the 4-DG ratio on the coloring of the MG solution.
[Figure 3] The left panel of Figure 3 is a graph showing the results of investigating the shinorine stabilizing effects of several compounds. The right panel of Figure 3 is a graph showing the results of investigating the MG stabilizing effects of several compounds constituting nucleic acids.
[Figure 4] The left panel of Figure 4 is a graph showing the results of examining the mycosporine-glycine-alanine (MGA) stabilizing effects of several bases constituting nucleic acids. The upper right panel of Figure 4 is a graph showing the results of examining the MG stabilizing effect (after 20 days) of several bases constituting nucleic acids. The lower right panel of Figure 4 is a graph showing the results of examining the MG stabilizing effects (after 49 days) of several compounds.

### [Description of embodiments]

In one aspect, the present invention provides a composition in which mycosporine-glycine (MG) is stabilized, wherein the composition comprises MG and is weakly acidic to weakly alkaline.

MG (Mycosporine-Glycine) is a compound having a structure below (Formula 1). MG is characterized by its high UV-A and UV-B absorption ability and excellent antioxidant function.

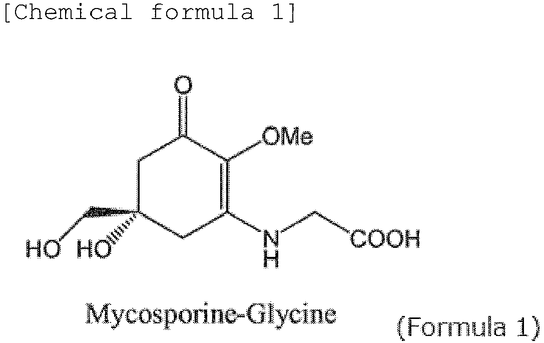

By making the pH of the composition of the present invention from weakly acidic to weakly alkaline, MG in the composition is stabilized and its decomposition is suppressed. Along with this, coloring of the composition can also be suppressed.

In the composition of this aspect, "MG is stabilized" means that decomposition of MG is suppressed. In determining the stabilization of MG, for example, the MG residual rate after a certain period of time may be used as an index. For example, in an experiment in which MG may be placed in a composition at 60°C for 4 days, the MG residual rate is about 80% or more, preferably about 84% or more, more preferably about 88% or more after 4 days, compared to that at the start of the experiment.

"The pH of the composition is weakly acidic to weakly alkaline" means, for example, that the pH is about 5.0 to about 9.0. Preferably, the pH may be about 5.4 to about 8.8, such as about 5.4 to about 8.0, about 5.9 to about 8.8, about 5.9 to about 8.0, etc.

For example, the pH of the composition can be maintained at a desired value by using known buffers.

In this specification, when "about" is added before a numerical value, it means a range of ±20% of the numerical value, preferably ±10% of the numerical value, and more preferably ±5% of the numerical value.

The concentration of MG in the composition of this aspect may be arbitrary.

The form of the above composition is typically a solution, but it may also be a semi-solid such as cream or pasta, or a solid such as a gel. In the case of a solution, the solvent may be water, an organic solvent such as ethanol, or a mixture of water and an organic solvent. The composition may be, for example, a cosmetic composition (for example, a sunscreen cream, lotion, whitening cosmetic, etc.), a skin composition (for example, a liquid, a spray, an ointment, a cream, a pasta, a gel, a patch, etc.), etc. Bases, carriers, and excipients for producing these compositions are known.

The above composition may contain components other than MG, such as pH adjusters (buffers), salts, fragrances, colorants, preservatives, or compounds that improve the stabilization of MG (described below).

In another aspect, the present invention provides a method for stabilizing MG, which comprises subjecting MG to weakly acidic to weakly alkaline conditions.

Stabilization of MG, from weakly acidic to weakly alkaline, such a pH range are as explained above.

Subjecting MG under weakly acidic to weakly alkaline conditions typically means mixing MG in a neutral or weakly alkaline solution composition, but MG may be mixed into a weakly acidic to weakly alkaline semi-solid composition or a solid composition. For example, by using a known buffer, conditions from weakly acidic to weakly alkaline can be created.

The present inventors have found for the first time a range of content of 4-DG in a composition that can stabilize MG in the composition. Accordingly, in further aspect, the present invention provides a composition in which MG is stabilized, wherein the composition comprises:
(a) MG, and
(b) 10 parts by weight or less of 4-deoxygadusol (4-DG).

4-DG (4-deoxygadusol) is a compound having the structure shown below (Formula 2):

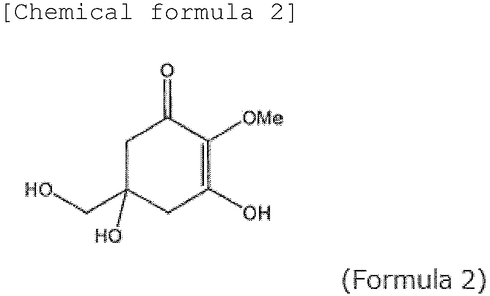

The amount of 4-DG (sometimes referred to as 4-DG ratio) in the composition of this aspect is expressed in parts by weight relative to MG. The amount of 4-DG in the composition of this embodiment may be any concentration that provides the desired MG stabilizing effect, such as about 12 parts by weight or less, preferably about 11 parts by weight or less, more preferably about 10 parts by weight or less. The amount may be, for example, about 0.5 parts by weight to about 12 parts by weight, about 1.0 parts by weight to about 11 parts by weight, about 1.5 parts by weight to about 10 parts by weight, etc.

The pH of the composition of this aspect is preferably from weakly acidic to weakly alkaline, and such a pH range is as explained above.

In the composition of this aspect, "MG is stabilized" means that decomposition of MG is suppressed. In determining the stabilization of MG, for example, the MG residual rate after a certain period of time may be used as an index. For example, in an experiment in which MG is placed in a composition at 60°C and pH 7 for 57 days, the MG residual rate may be about 71% or more, preferably about 73% or more, more preferably about 75% or more after 57 days compared to that at the start of the experiment.

The composition is as explained above.

The concentration of MG in the composition of this aspect may be arbitrary.

In yet another aspect, the present invention provides a method for stabilizing MG, comprising mixing MG with 10 parts by weight or less of 4-DG.

4-DG in an amount of 10 parts by weight or less, and the stabilization of MG are as explained above. 4-DG and MG may be mixed in a solution. 4-DG and MG may be kneaded in a semi-solid material such as cream or pasta. Alternatively, a mixed solution of 4-DG and MG may be gelled by a known method.

The present inventors have found for the first time that MAA in the composition can be stabilized by adding one or more compounds selected from a base constituting a nucleic acid, a nucleoside, glycine, and tranexamic acid to the composition containing MAA. Accordingly, in yet another aspect, the invention provides a composition in which MAA is stabilized, wherein the composition comprises:
(a) mycosporine-like amino acid (MAA), and
(b) one or more selected from a base constituting a nucleic acid, a nucleoside, glycine, and tranexamic acid.

MAA is a general term for compounds in which an amino acid is bonded to a cyclohexenone or cyclohexenimine skeleton that may have a substituent.

MAAs include, but are not limited to, for example, shinorine (Formula 3), porphyra-334 (Formula 4), asterina-330 (Formula 5), palythene (Formula 6), palythine (Formula 7), mycosporine-glycine (Formula 8), mycosporine-glycine-valine (Formula 9), mycosporine-glycine-alanine (Formula 10), mycosporine serinol (Formula 11), and the like.

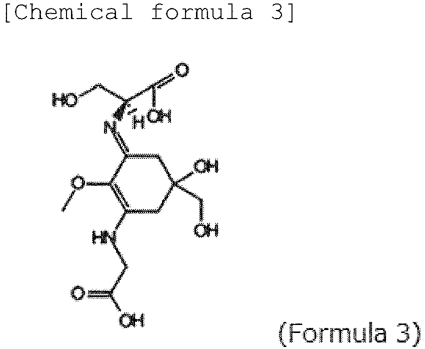

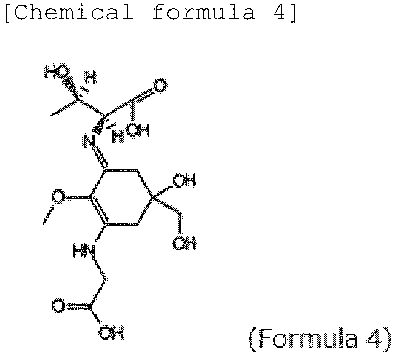

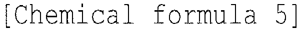

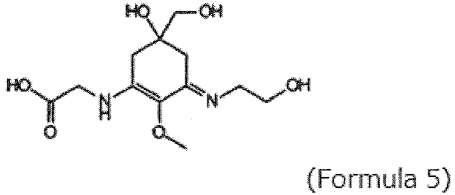

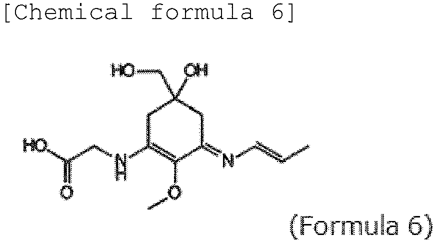

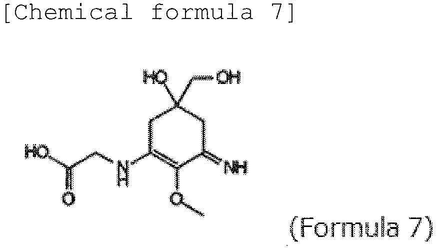

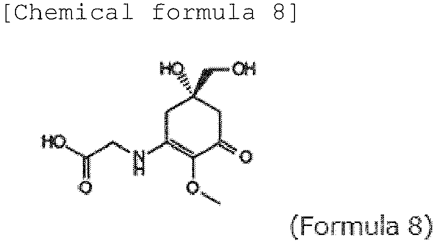

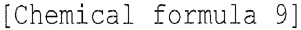

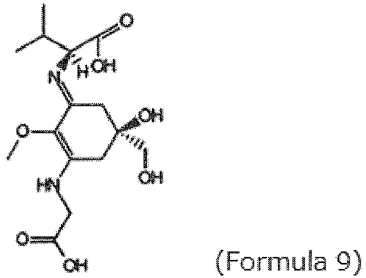

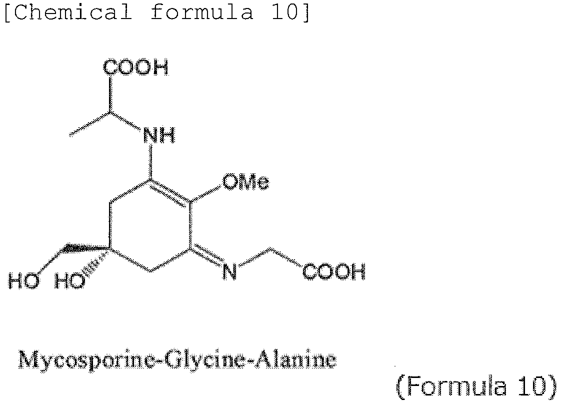

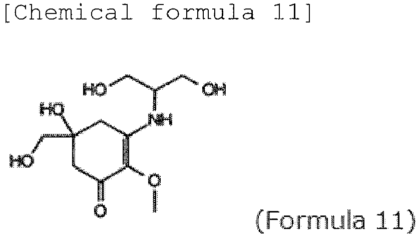

The MAA stabilized in the composition of this aspect may be any MAA. Preferred MAAs include, but are not limited to, MG, shinorine, mycosporine-glycine-alanine, and porphyra-334. One type of MAA may be stabilized, or two or more types of MAA may be stabilized.

The MAA stabilizer used in the composition of this aspect is selected from a base constituting a nucleic acid, a nucleoside, glycine, and tranexamic acid. One type of MAA stabilizer may be used, or two or more types of MAA stabilizer may be used.

Bases constituting nucleic acids include purine bases (adenine, guanine) and pyrimidine bases (cytosine, uracil, thymine). In this specification, in addition to the above, other bases constituting nucleic acids include hypoxanthine, xanthine, 7-methylguanine, 5,6-dihydrouracil, 5-methylcytosine, 5-hydroxymethylcytosine, etc. An example of a preferred base constituting a nucleic acid is adenine, but is not limited thereto.

Glycine is a known natural amino acid.

Tranexamic acid is a known artificial amino acid represented by Formula 12, and is used as a hemostatic agent and an anti-inflammatory agent.

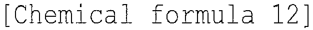

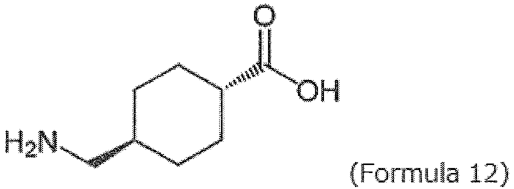

MAA stabilizers may be nucleosides. Examples of bases constituting a nucleoside include, but are not limited to, purine bases such as adenine and guanine, pyrimidine bases such as thymine, cytosine, and uracil, nicotinamide, and dimethylisoalloxazine. Examples of sugars constituting a nucleoside include, but are not limited to, ribose and deoxyribose. Nucleosides that can be used as MAA stabilizers include, but are not limited to, adenosine, guanosine, uridine, cytidine, 5-methyluridine, deoxyadenosine, deoxyguanosine, deoxyuridine, deoxycytidine, thymidine, and the like.

The pH of the composition of this aspect is preferably from weakly acidic to weakly alkaline, and such a pH range is as explained above. The composition is also as explained above.

In the composition of this aspect, "MAA is stabilized" means that decomposition of MAA is suppressed. In determining MAA stabilization, for example, the MAA residual rate after a certain period of time may be used as an index. For example, in an experiment where MG is left at 60°C and pH 7.5 for 60 days, the MG residual rate after 60 days is about 68% or more, preferably about 72% or more, more preferably about 76% or more, compared to that at the start of the experiment. Further, for example, in an experiment where shinorine is left at 60° C and pH 7.5 for 3 days, the residual rate of shinorine is about 64.5% or more after 3 days, preferably about 65.5% or more, more preferably about 66.5% or more compared to that at the start of the experiment.

The concentration of MAA in the composition of this aspect may be arbitrary.

The concentration of the MAA stabilizer in the composition of this aspect may be any concentration that provides the desired MAA stabilizing effect, and may be determined by taking into account the MAA concentration in the composition. The concentration of the MAA stabilizer may be, for example, about 0.05% to about 1% by weight, about 0.1% to about 0.5% by weight, about 0.1% to about 0.2% by weight, etc.

In yet another aspect, the present invention provides a method for stabilizing MAA, comprising mixing MAA with one or more selected from a base constituting a nucleic acid, a nucleoside, glycine, and tranexamic acid.

Preferred MAA in this method include, but are not limited to, MG, shinorine, mycosporine-glycine-alanine, and porphyra-334. An example of a base constituting the nucleic acid preferably used in this method is adenine, but is not limited thereto.

The present invention is explained in detail and specifically by way of Examples below, however the Examples are not intended to limit the scope of the present invention.

### [Example 1]

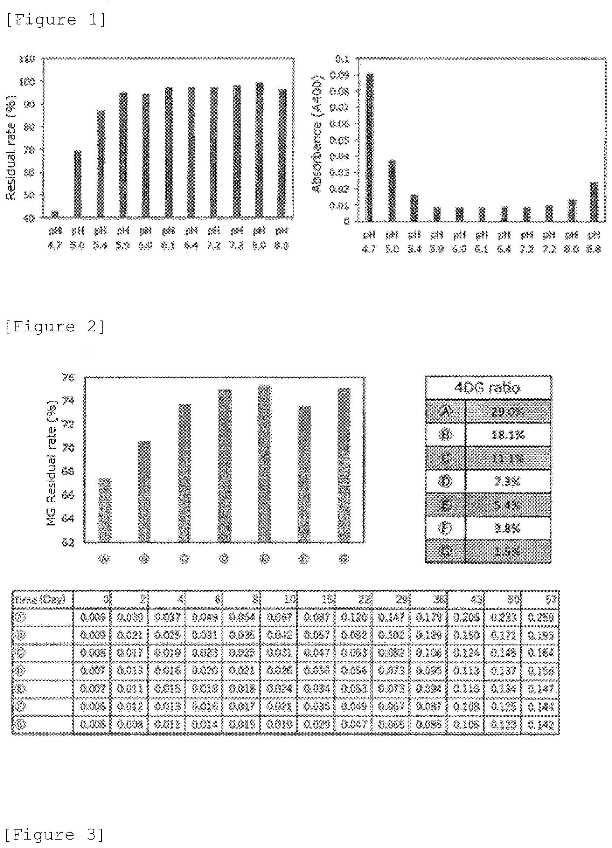

Influence of pH on the stability of MG in a solution and the coloring of an MG solution were investigated. Adjustment of the solution to each pH was performed using 0.1 M phosphate buffer and phosphoric acid and NaOH as necessary. The MG concentration in the solution was 0.1%. MG solutions of each pH were prepared and left at 60° C for 4 days, and then the amount of MG and the absorbance (400 nm) of the solutions were measured. The MG residual rate was determined by comparing with the amount at the start of the experiment. The results are shown in Figure 1.

It was found that the MG residual rate increased and MG was stabilized in weakly acidic to weakly alkaline conditions. Furthermore, it was found that coloring of the MG solution was also suppressed in weakly acidic to weakly alkaline conditions. The MG residual rate was 88% or more at pH 5.4 to 8.8. From these results, it was shown that the stability of MG becomes extremely high in weakly acidic to weakly alkaline conditions. The absorbance of the solution was very low (0.018 or less) at pH 5.4 to 8.0.

### [Example 2]

The effect of 4-DG on stabilizing MG was investigated. Solutions at pH 7 (in 0.1M phosphate buffer) containing various ratios of 4-DG to MG (0.1%) were prepared, and the amount of MG after standing at 60°C for 57 days was measured. Then, the MG residual rate was determined. Additionally, the absorbance (400 nm) of the solution was measured over time during the experiment period. The results are shown in Figure 2.

When the 4-DG ratio was 1.5 parts by weight to 11.1 parts by weight, the residual rate of MG was as high as 74% or more, indicating that MG was stabilized for a long time. Further, when the 4-DG ratio was 1.5 parts by weight to 11.1 parts by weight, the absorbance of the solution was 0.17 or less on the 57th day, and the coloration suppressing effect of the solution was also high.

### [Example 3]

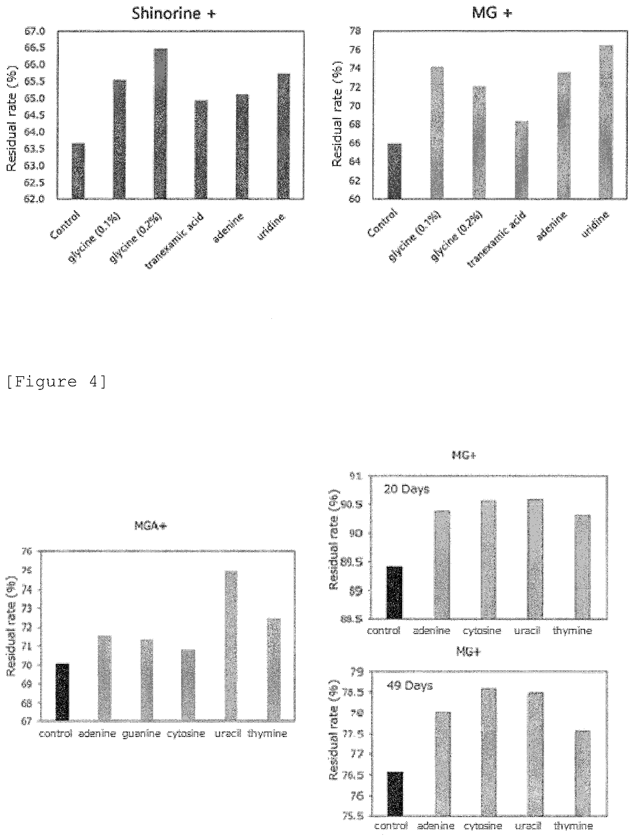

The effects of glycine, tranexamic acid, adenine, and uridine on the stability of shinorine and MG were investigated. A 0.1% shinorine solution (adjusted to pH 7.5 with 0.1M phosphate buffer) containing glycine (0.1%, 0.2%), tranexamic acid (0.2%), adenine (0.1%), or uridine (0.2%) was prepared. A 0.15% MG solution (adjusted to pH 7.5 with 0.1M phosphate buffer) containing glycine (0.1%, 0.2%), tranexamic acid (0.2%), adenine (0.1%), or uridine (0.2%) was prepared. After these solutions were left at 60° C for 3 days for shinorine and 60 days for MG, the amounts of shinorine and MG were measured to determine the residual rate. The results are shown in Figure 3.

The stabilizing effects of glycine, tranexamic acid, adenine, and uridine on both shinorine and MG were confirmed.

### [Example 4]

The influence of bases (adenine, guanine, cytosine, uracil, and thymine) constituting nucleic acids on the stability of MGA and MG was investigated. A 0.1% MGA solution (adjusted to pH 7.5 with 0.1M phosphate buffer) each containing adenine (0.01%), guanine (0.2%), cytosine (0.2%), uracil (0.2%), or thymine (0.2%) was prepared. A 0.1% MG solution (adjusted to pH 7.5 with 0.1M phosphate buffer) each containing adenine (0.01%), cytosine (0.2%), uracil (0.2%), or thymine (0.2%) was prepared. After these solutions were left at 60° C for 3 days for MGA, 20 days and 49 days for MG, the amounts of MGA and MG were measured to determine the residual rate. The results are shown in Figure 4.

The stabilizing effects of adenine, guanine, cytosine, uracil, and thymine on MGA were confirmed. The stabilizing effects of adenine, cytosine, uracil, and thymine on MG were also confirmed.

### [Industrial applicability]

The present invention provides compositions and methods for stabilizing MAA. Therefore, it is useful in fields such as cosmetics and pharmaceuticals. The present invention is useful in the manufacture of cosmetics, skin external preparations, etc. for the purpose of UV protection, whitening, or antioxidation.

## Claims

1. A composition in which mycosporine-glycine (MG) is stabilized, wherein the composition comprises MG and is weakly acidic to weakly alkaline.

2. The composition of claim 1, pH of which is 5.4 to 8.0.

3. A method for stabilizing MG, comprising subjecting MG under weakly acidic to weakly alkaline conditions.

4. The method of claim 3, wherein pH is 5.4 to 8.0.

5. A composition in which MG is stabilized, wherein the composition comprises:
(a) MG, and
(b) 10 parts by weight or less of 4-deoxygadusol (4-DG).

6. The composition of claim 5, comprising 1.5 parts to 10 parts by weight of 4-DG.

7. The composition of claim 5 or 6, which is weakly acidic to weakly alkaline.

8. The composition of claim 7, pH of which is 5.4 to 8.0.

9. A method for stabilizing MG, comprising mixing MG with 10 parts by weight or less of 4-DG.

10. A composition in which MAA is stabilized, wherein the composition comprises:
(a) mycosporine-like amino acid (MAA), and
(b) one or more selected from a base constituting a nucleic acid, a nucleoside, glycine, and tranexamic acid.

11. The composition of claim 10, wherein MAA is one or more MAA selected from MG, shinorine, mycosporine-glycine-alanine, and porphyra-334.

12. The composition of claim 10 or 11, wherein MAA is one or more MAA selected from MG, mycosporine-glycine-alanine, and shinorine.

13. The composition of any one of claims 10-12, which is weakly acidic to weakly alkaline.

14. The composition of claim 13, pH of which is 5.4 to 8.0.

15. A method for stabilizing MAA, comprising mixing MAA with one or more selected from a base constituting a nucleic acid, a nucleoside, glycine, and tranexamic acid.

16. The method of claim 15, wherein MAA is one or more MAA selected from MG, shinorine, mycosporine-glycine-alanine, and porphyra-334.

17. The method of claim 15 or 16, wherein MAA is one or more MAA selected from MG, mycosporine-glycine-alanine, and shinorine.
